# EUROPEAN PATENT APPLICATION

(11) **EP 1 705 487 A2**
(43) Date of publication of application: **27.09.2006**
(21) Application number: 06011486.5
(22) Date of filing: 17.07.2001
(51) Int. Cl.: G01N 33/574, C07K 16/18, C07K 16/30

(54) **Method of diagnosing, monitoring, staging, imaging and treating colon cancer**

(30) Priority: 17.07.2000 US 618596
(62) Divisional of application: 01984249.1
(71) Applicant: Diadexus, Inc., South San Francisco, CA 94080 (US)
(72) Inventor: Macina, Roberto A., San Jose, CA 95128 (US); Sun, Yongming, San Jose, CA 95128 (US)
(74) Representative: Peebles, Katrina

(57) **Abstract**

The present invention provides a method of diagnosing, monitoring, staging, imaging and treating colon cancer.

SEQUENCE LISTING

<110> Macina, Roberto A.
      Sun, Yongming
      diaDexus, Inc.

<120> Method of Diagnosing, Monitoring, Staging, Imaging and
      Treating Colon Cancer

<130> DEX-0213

<140>
<141>

<150> 09/618,596
<151> 2000-07-17

<160> 84

<170> PatentIn Ver. 2.0

<210> 1
<211> 1881
<212> DNA
<213> Homo sapiens

<400> 1
ggacctctcc agaatccgga ttgctgaatc ttccctgttg cctagaaggg ctccaaacca 60
cctcttgaca atgggaaact gggtggttaa ccactggttt tcagttttgt ttctggttgt 120
ttggttaggg ctgaatgttt tcctgtttgt ggatgccttc ctgaaatatg agaaggccga 180
caaatactac tacacaagaa aaatccttgg gtcaacattg gcctgtgccc gagcgtctgc 240
tctctgcttg aattttaaca gcacgctgat cctgcttcct gtgtgtcgca atctgctgtc 300
cttcctgagg ggcacctgct cattttgcag ccgcacactg agaaagcaat tggatcacaa 360
cctcaccttc cacaagctgg tggcctatat gatctgccta catacagcta ttcacatcat 420
tgcacacctg tttaactttg actgctatag cagaagccga caggccacag atggctccct 480
tgcctccatt ctctccagcc tatctcatga tgagaaaaag gggggttctt ggctaaatcc 540
catccagtcc cgaaacacga cagtggagta tgtgacattc accagcattg ctggtctcac 600
tggagtgatc atgacaatag ccttgattct catggtaact tcagctactg agttcatccg 660
gaggagttat tttgaagtct tctggtatac tcaccacctt tttatcttct atatccttgg 720
cttagggatt cacggcattg gtggaattgt ccggggtcaa acagaggaga gcatgaatga 780
gagtcatcct cgcaagtgtg cagagtcttt tgagatgtgg gatgatcgtg actcccactg 840
taggcgccct aagtttgaag ggcatccccc tgagtcttgg aagtggatcc ttgcaccggt 900
cattctttat atctgtgaaa ggatcctccg gttttaccgc tcccagcaga aggttgtgat 960
taccaaggtt gttatgcacc catccaaagt tttggaattg cagatgaaca agcgtggctt 1020
cagcatggaa gtggggcagt atatctttgt taattgcccc tcaatctctc tcctggaatg 1080
gcatcctttt actttgacct ctgctccaga ggaagatttc ttctccattc atatccgagc 1140
agcaggggac tggacagaaa atctcataag ggctttcgaa caacaatatt caccaattcc 1200
caggattgaa gtggatggtc cctttggcac agccagtgag gatgttttcc agtatgaagt 1260

ggctgtgctg gttggagcag gaattggggt cacccccttt gcttctatct tgaaatccat 1320
ctggtacaaa ttccagtgtg cagaccacaa cctcaaaaca aaaaagatct atttctactg 1380
gatctgcagg gagacaggtg ccttttcctg gttcaacaac ctgttgactt ccctggaaca 1440
ggagatggag gaattaggca aagtgggttt tctaaactac cgtctcttcc tcaccggatg 1500
ggacagcaat attgttggtc atgcagcatt aaactttgac aaggccactg acatcgtgac 1560
aggtctgaaa cagaaaacct cctttgggag accaatgtgg gacaatgagt tttctacaat 1620
agctacctcc caccccaagt ctgtagtggg agttttctta tgtggccctc ggactttggc 1680
aaagagcctg cgcaaatgct gtcaccgata ttccagtctg gatcctagaa aggttcaatt 1740
ctacttcaac aaagaaaatt tttgagttat aggaataagg acggtaatct gcattttgtc 1800
tctttgtatc ttcagtaatt tacttggtct cgtcaggttt gagcagtcac tttaggataa 1860
gaatgtgcct ctcaagcctt g 1881

<210> 2
<211> 658
<212> DNA
<213> Homo sapiens

<400> 2
ggacctctcc agaatccgga ttgctgaatc ttccctgttg cctagaaggg ctccaaacca 60
cctcttgaca atgggaaact gggtggttaa ccactggttt tcagttttgt ttctggttgt 120
ttggttaggg ctgaatgttt tcctgtttgt ggatgccttc ctgaaatatg agaaggccga 180
caaatactac tacacaagaa aaatccttgg gtcaacattg gcctgtgccc gagcgtctgc 240
tctctgcttg aattttaaca gcacgctgat cctgcttcct gtgtgtcgca atctgctgtc 300
cttcctgagg ggcacctgct cattttgcag ccgcacactg agaaagcaat tggatcacaa 360
cctcaccttc cacaagctgg tggcctatat gatctgccta catacagcta ttcacatcat 420
tgcacacctg tttaactttg actgctatag cagaagccga caggccacag atggctccct 480
tgcctccatt ctctccagcc tatctcatga tgagaaaaag gggggttctt ggctaaatcc 540
catccatccc catataacac caacagtgta catgtttact gtcacttttg atatggtctt 600
atccagtgtg aacagcaatt tattattttt gctcatcaaa aaataaagga ttttcttc 658

<210> 3
<211> 564
<212> PRT
<213> Homo sapiens

<400> 3
Met Gly Asn Trp Val Val Asn His Trp Phe Ser Val Leu Phe Leu Val
1               5                   10                  15

Val Trp Leu Gly Leu Asn Val Phe Leu Phe Val Asp Ala Phe Leu Lys
            20                  25                  30

Tyr Glu Lys Ala Asp Lys Tyr Tyr Tyr Thr Arg Lys Ile Leu Gly Ser
        35                  40                  45

Thr Leu Ala Cys Ala Arg Ala Ser Ala Leu Cys Leu Asn Phe Asn Ser
    50                  55                  60

Thr Leu Ile Leu Leu Pro Val Cys Arg Asn Leu Leu Ser Phe Leu Arg
65                  70                  75                  80

Gly Thr Cys Ser Phe Cys Ser Arg Thr Leu Arg Lys Gln Leu Asp His
                85                  90                  95

Asn Leu Thr Phe His Lys Leu Val Ala Tyr Met Ile Cys Leu His Thr
            100                 105                 110

Ala Ile His Ile Ile Ala His Leu Phe Asn Phe Asp Cys Tyr Ser Arg
        115                 120                 125

Ser Arg Gln Ala Thr Asp Gly Ser Leu Ala Ser Ile Leu Ser Ser Leu
    130                 135                 140

Ser His Asp Glu Lys Lys Gly Gly Ser Trp Leu Asn Pro Ile Gln Ser
145                 150                 155                 160

Arg Asn Thr Thr Val Glu Tyr Val Thr Phe Thr Ser Val Ala Gly Leu
                165                 170                 175

Thr Gly Val Ile Met Thr Ile Ala Leu Ile Leu Met Val Thr Ser Ala
            180                 185                 190

Thr Glu Phe Ile Arg Arg Ser Tyr Phe Glu Val Phe Trp Tyr Thr His
        195                 200                 205

His Leu Phe Ile Phe Tyr Ile Leu Gly Leu Gly Ile His Gly Ile Gly
    210                 215                 220

Gly Ile Val Arg Gly Gln Thr Glu Glu Ser Met Asn Glu Ser His Pro
225                 230                 235                 240

Arg Lys Cys Ala Glu Ser Phe Glu Met Trp Asp Asp Arg Asp Ser His
                245                 250                 255

Cys Arg Arg Pro Lys Phe Glu Gly His Pro Pro Glu Ser Trp Lys Trp
            260                 265                 270

Ile Leu Ala Pro Val Ile Leu Tyr Ile Cys Glu Arg Ile Leu Arg Phe
        275                 280                 285

Tyr Arg Ser Gln Gln Lys Val Val Ile Thr Lys Val Val Met His Pro
    290                 295                 300

Ser Lys Val Leu Glu Leu Gln Met Asn Lys Arg Gly Phe Ser Met Glu
305                 310                 315                 320

Val Gly Gln Tyr Ile Phe Val Asn Cys Pro Ser Ile Ser Leu Leu Glu
                325                 330                 335

Trp His Pro Phe Thr Leu Thr Ser Ala Pro Glu Glu Asp Phe Phe Ser
            340                 345                 350

Ile His Ile Arg Ala Ala Gly Asp Trp Thr Glu Asn Leu Ile Arg Ala
        355                 360                 365

Phe Glu Gln Gln Tyr Ser Pro Ile Pro Arg Ile Glu Val Asp Gly Pro
    370                 375                 380

Phe Gly Thr Ala Ser Glu Asp Val Phe Gln Tyr Glu Val Ala Val Leu
385                 390                 395                 400

Val Gly Ala Gly Ile Gly Val Thr Pro Phe Ala Ser Ile Leu Lys Ser
                405                 410                 415

Ile Trp Tyr Lys Phe Gln Cys Ala Asp His Asn Leu Lys Thr Lys Lys
            420                 425                 430

Ile Tyr Phe Tyr Trp Ile Cys Arg Glu Thr Gly Ala Phe Ser Trp Phe
        435                 440                 445

Asn Asn Leu Leu Thr Ser Leu Glu Gln Glu Met Glu Glu Leu Gly Lys
    450                 455                 460

Val Gly Phe Leu Asn Tyr Arg Leu Phe Leu Thr Gly Trp Asp Ser Asn
465                 470                 475                 480

Ile Val Gly His Ala Ala Leu Asn Phe Asp Lys Ala Thr Asp Ile Val
                485                 490                 495

Thr Gly Leu Lys Gln Lys Thr Ser Phe Gly Arg Pro Met Trp Asp Asn
            500                 505                 510

Glu Phe Ser Thr Ile Ala Thr Ser His Pro Lys Ser Val Val Gly Val
        515                 520                 525

Phe Leu Cys Gly Pro Arg Thr Leu Ala Lys Ser Leu Arg Lys Cys Cys
    530                 535                 540

His Arg Tyr Ser Ser Leu Asp Pro Arg Lys Val Gln Phe Tyr Phe Asn
545                 550                 555                 560

Lys Glu Asn Phe

<210> 4
<211> 191
<212> PRT
<213> Homo sapiens

<400> 4
Met Gly Asn Trp Val Val Asn His Trp Phe Ser Val Leu Phe Leu Val
1               5                   10                  15

Val Trp Leu Gly Leu Asn Val Phe Leu Phe Val Asp Ala Phe Leu Lys
            20                  25                  30

Tyr Glu Lys Ala Asp Lys Tyr Tyr Tyr Thr Arg Lys Ile Leu Gly Ser
        35                  40                  45

Thr Leu Ala Cys Ala Arg Ala Ser Ala Leu Cys Leu Asn Phe Asn Ser
    50                  55                  60

Thr Leu Ile Leu Leu Pro Val Cys Arg Asn Leu Leu Ser Phe Leu Arg
65                  70                  75                  80

Gly Thr Cys Ser Phe Cys Ser Arg Thr Leu Arg Lys Gln Leu Asp His
                85                  90                  95

Asn Leu Thr Phe His Lys Leu Val Ala Tyr Met Ile Cys Leu His Thr
            100                 105                 110

Ala Ile His Ile Ile Ala His Leu Phe Asn Phe Asp Cys Tyr Ser Arg
        115                 120                 125

Ser Arg Gln Ala Thr Asp Gly Ser Leu Ala Ser Ile Leu Ser Ser Leu
    130                 135                 140

Ser His Asp Glu Lys Lys Gly Gly Ser Trp Leu Asn Pro Ile His Pro
145                 150                 155                 160

His Ile Thr Pro Thr Val Tyr Met Phe Thr Val Thr Phe Asp Met Val
                165                 170                 175

Leu Ser Ser Val Asn Ser Asn Leu Leu Phe Leu Leu Ile Lys Lys
            180                 185                 190

<210> 5
<211> 5
<212> PRT
<213> Homo sapiens

<400> 5
Leu Cys Leu Asn Phe
1               5

<210> 6
<211> 4
<212> PRT
<213> Homo sapiens

<400> 6
Asn Ser Thr Leu
1

<210> 7
<211> 5
<212> PRT
<213> Homo sapiens

<400> 7
Ile Leu Leu Pro Val
1               5

<210> 8
<211> 5
<212> PRT
<213> Homo sapiens

<400> 8
Lys Gln Leu Asp His
1               5

<210> 9
<211> 4
<212> PRT
<213> Homo sapiens

<400> 9
Asn Leu Thr Phe
1

<210> 10
<211> 5

<212> PRT
<213> Homo sapiens

<400> 10
His Lys Leu Val Ala
1               5

<210> 11
<211> 5
<212> PRT
<213> Homo sapiens

<400> 11
Pro Ile Gln Ser Arg
1               5

<210> 12
<211> 4
<212> PRT
<213> Homo sapiens

<400> 12
Asn Thr Thr Val
1

<210> 13
<211> 5
<212> PRT
<213> Homo sapiens

<400> 13
Glu Tyr Val Thr Phe
1               5

<210> 14
<211> 5
<212> PRT
<213> Homo sapiens

<400> 14
Thr Glu Glu Ser Met
1               5

<210> 15
<211> 4
<212> PRT
<213> Homo sapiens

<400> 15
Asn Glu Ser His
1

<210> 16
<211> 5
<212> PRT
<213> Homo sapiens

<400> 16
Pro Arg Lys Cys Ala
1               5

<210> 17
<211> 5
<212> PRT
<213> Homo sapiens

<400> 17
Ile Leu Ser Ser Leu
1               5

<210> 18
<211> 4
<212> PRT
<213> Homo sapiens

<400> 18
Ser His Asp Glu
1

<210> 19
<211> 5
<212> PRT
<213> Homo sapiens

<400> 19
Lys Lys Gly Gly Ser
1               5

<210> 20
<211> 5
<212> PRT
<213> Homo sapiens

<400> 20
Ile Gln Ser Arg Asn
1               5

<210> 21
<211> 4
<212> PRT
<213> Homo sapiens

<400> 21
Thr Thr Val Glu
1

<210> 22
<211> 5
<212> PRT
<213> Homo sapiens

<400> 22
Tyr Val Thr Phe Thr
1               5

<210> 23
<211> 5
<212> PRT
<213> Homo sapiens

<400> 23
Ile Leu Met Val Thr
1               5

<210> 24
<211> 4
<212> PRT
<213> Homo sapiens

<400> 24

Ser Ala Thr Glu
1

<210> 25
<211> 5
<212> PRT
<213> Homo sapiens

<400> 25
Phe Ile Arg Arg Ser
1               5

<210> 26
<211> 5
<212> PRT
<213> Homo sapiens

<400> 26
Glu Phe Ile Arg Arg
1               5

<210> 27
<211> 4
<212> PRT
<213> Homo sapiens

<400> 27
Ser Tyr Phe Glu
1

<210> 28
<211> 5
<212> PRT
<213> Homo sapiens

<400> 28
Val Phe Trp Tyr Thr
1               5

<210> 29
<211> 5
<212> PRT
<213> Homo sapiens

<400> 29
Gly Gln Thr Glu Glu
1               5

<210> 30
<211> 4
<212> PRT
<213> Homo sapiens

<400> 30
Ser Met Asn Glu
1

<210> 31
<211> 5
<212> PRT
<213> Homo sapiens

<400> 31
Ser His Pro Arg Lys
1               5

<210> 32
<211> 5
<212> PRT
<213> Homo sapiens

<400> 32
Asn Cys Pro Ser Ile
1               5

<210> 33
<211> 4
<212> PRT
<213> Homo sapiens

<400> 33
Ser Leu Leu Glu
1

<210> 34
<211> 5

<212> PRT
<213> Homo sapiens

<400> 34
Trp His Pro Phe Thr
1               5


<210> 35
<211> 5
<212> PRT
<213> Homo sapiens

<400> 35
Pro Phe Thr Leu Thr
1               5


<210> 36
<211> 4
<212> PRT
<213> Homo sapiens

<400> 36
Ser Ala Pro Glu
1


<210> 37
<211> 5
<212> PRT
<213> Homo sapiens

<400> 37
Glu Asp Phe Phe Ser
1               5


<210> 38
<211> 5
<212> PRT
<213> Homo sapiens

<400> 38
Asp Gly Pro Phe Gly
1               5

<210> 39
<211> 4
<212> PRT
<213> Homo sapiens

<400> 39
Thr Ala Ser Glu
1

<210> 40
<211> 5
<212> PRT
<213> Homo sapiens

<400> 40
Asp Val Phe Gln Tyr
1               5

<210> 41
<211> 5
<212> PRT
<213> Homo sapiens

<400> 41
Phe Asn Asn Leu Leu
1               5

<210> 42
<211> 4
<212> PRT
<213> Homo sapiens

<400> 42
Thr Ser Leu Glu
1

<210> 43
<211> 5
<212> PRT
<213> Homo sapiens

<400> 43
Gln Glu Met Glu Glu
1               5

<210> 44
<211> 5
<212> PRT
<213> Homo sapiens

<400> 44
Tyr Arg Leu Phe Leu
1               5

<210> 45
<211> 4
<212> PRT
<213> Homo sapiens

<400> 45
Thr Gly Trp Asp
1

<210> 46
<211> 5
<212> PRT
<213> Homo sapiens

<400> 46
Ser Asn Ile Val Gly
1               5

<210> 47
<211> 5
<212> PRT
<213> Homo sapiens

<400> 47
Cys Cys His Arg Tyr
1               5

<210> 48
<211> 4
<212> PRT
<213> Homo sapiens

<400> 48

Ser Ser Leu Asp
1

<210> 49
<211> 5
<212> PRT
<213> Homo sapiens

<400> 49
Pro Arg Lys Val Gln
1               5

<210> 50
<211> 5
<212> PRT
<213> Homo sapiens

<400> 50
Thr Arg Lys Ile Leu
1               5

<210> 51
<211> 6
<212> PRT
<213> Homo sapiens

<400> 51
Gly Ser Thr Leu Ala Cys
1               5

<210> 52
<211> 5
<212> PRT
<213> Homo sapiens

<400> 52
Ala Arg Ala Ser Ala
1               5

<210> 53
<211> 5
<212> PRT
<213> Homo sapiens

<400> 53
Arg Gln Ala Thr Asp
1               5

<210> 54
<211> 6
<212> PRT
<213> Homo sapiens

<400> 54
Gly Ser Leu Ala Ser Ile
1               5

<210> 55
<211> 5
<212> PRT
<213> Homo sapiens

<400> 55
Leu Ser Ser Leu Ser
1               5

<210> 56
<211> 5
<212> PRT
<213> Homo sapiens

<400> 56
Val Ala Gly Leu Thr
1               5

<210> 57
<211> 6
<212> PRT
<213> Homo sapiens

<400> 57
Gly Val Ile Met Thr Ile
1               5

<210> 58
<211> 5

<212> PRT
<213> Homo sapiens

<400> 58
Ala Leu Ile Leu Met
1               5


<210> 59
<211> 5
<212> PRT
<213> Homo sapiens

<400> 59
Ile His Gly Ile Gly
1               5


<210> 60
<211> 6
<212> PRT
<213> Homo sapiens

<400> 60
Gly Ile Val Arg Gly Gln
1               5


<210> 61
<211> 5
<212> PRT
<213> Homo sapiens

<400> 61
Thr Glu Glu Ser Met
1               5


<210> 62
<211> 5
<212> PRT
<213> Homo sapiens

<400> 62
Val Ala Val Leu Val
1               5

<210> 63
<211> 6
<212> PRT
<213> Homo sapiens

<400> 63
Gly Ala Gly Ile Gly Val
1               5

<210> 64
<211> 5
<212> PRT
<213> Homo sapiens

<400> 64
Thr Pro Phe Ala Ser
1               5

<210> 65
<211> 5
<212> PRT
<213> Homo sapiens

<400> 65
Pro Lys Ser Val Val
1               5

<210> 66
<211> 6
<212> PRT
<213> Homo sapiens

<400> 66
Gly Val Phe Leu Cys Gly
1               5

<210> 67
<211> 5
<212> PRT
<213> Homo sapiens

<400> 67
Pro Arg Thr Leu Ala
1               5

<210> 68
<211> 5
<212> PRT
<213> Homo sapiens

<400> 68
Asp Lys Tyr Tyr Tyr
1               5


<210> 69
<211> 5
<212> PRT
<213> Homo sapiens

<400> 69
Ile Leu Gly Ser Thr
1               5


<210> 70
<211> 5
<212> PRT
<213> Homo sapiens

<400> 70
Ser Phe Cys Ser Arg
1               5


<210> 71
<211> 5
<212> PRT
<213> Homo sapiens

<400> 71
Lys Gln Leu Asp His
1               5


<210> 72
<211> 5
<212> PRT
<213> Homo sapiens

<400> 72

Gly His Pro Pro Glu
1               5

<210> 73
<211> 5
<212> PRT
<213> Homo sapiens

<400> 73
Trp Ile Leu Ala Pro
1               5

<210> 74
<211> 5
<212> PRT
<213> Homo sapiens

<400> 74
Asp His Asn Leu Lys
1               5

<210> 75
<211> 5
<212> PRT
<213> Homo sapiens

<400> 75
Ile Tyr Phe Tyr Trp
1               5

<210> 76
<211> 5
<212> PRT
<213> Homo sapiens

<400> 76
Arg Thr Leu Ala Lys
1               5

<210> 77
<211> 5
<212> PRT
<213> Homo sapiens

<400> 77
Lys Cys Cys His Arg
1               5

<210> 78
<211> 5
<212> PRT
<213> Homo sapiens

<400> 78
Thr Glu Phe Ile Arg
1               5

<210> 79
<211> 9
<212> PRT
<213> Homo sapiens

<400> 79
Arg Ser Tyr Phe Glu Val Phe Trp Tyr
1               5

<210> 80
<211> 5
<212> PRT
<213> Homo sapiens

<400> 80
Thr His His Leu Phe
1               5

<210> 81
<211> 5
<212> PRT
<213> Homo sapiens

<400> 81
Thr Glu Asn Leu Ile
1               5

<210> 82
<211> 7

<212> PRT
<213> Homo sapiens

<400> 82
Arg Ala Phe Glu Gln Gln Tyr
1               5



<210> 83
<211> 5
<212> PRT
<213> Homo sapiens

<400> 83
Ser Pro Ile Pro Arg
1               5



<210> 84
<211> 2608
<212> DNA
<213> Homo sapiens

<220>
<221> unsure
<222> (2025)

<220>
<221> unsure
<222> (2036)

<220>
<221> unsure
<222> (2163)

<220>
<221> unsure
<222> (2263)

<400> 84
gctgatagca cagttctgtc cagagaagga aggcggaata aacttattca ttcccaggaa 60
ctcttggggt aggtgtgtgt ttttcacatc ttaaaggctc acagaccctg cgctggacaa 120
atgttccatt cctgaaggac ctctccagaa tccggattgc tgaatcttcc ctgttgccta 180
gaagggctcc aaaccacctc ttgacaatgg gaaactgggt ggttaaccac tggttttcag 240
ttttgtttct ggttgtttgg ttagggctga atgttttcct gtttgtggat gccttcctga 300
aatatgagaa ggccgacaaa tactactaca caagaaaaat ccttgggtca acattggcct 360
gtgcccgagc gtctgctctc tgcttgaatt ttaacagcac gctgatcctg cttcctgtgt 420
gtcgcaatct gctgtccttc ctgaggggca cctgctcatt ttgcagccgc acactgagaa 480

agcaattgga tcacaacctc accttccaca agctggtggc ctatatgatc tgcctacata 540
cagctattca catcattgca cacctgttta actttgactg ctatagcaga agccgacagg 600
ccacagatgg ctcccttgcc tccattctct ccagcctatc tcatgatgag aaaaaggggg 660
gttcttggct aaatcccatc cagtcccgaa acacgacagt ggagtatgtg acattcacca 720
gcgttgctgg tctcactgga gtgatcatga caatagcctt gattctcatg gtaacttcag 780
ctactgagtt catccggagg agttattttg aagtcttctg gtatactcac caccttttca 840
tcttctatat ccttggctta gggattcacg gcattggtgg aattgtccgg ggtcaaacag 900
aggagagcat gaatgagagt catcctcgca agtgtgcaga gtcttttgag atgtgggatg 960
atcgtgactc ccactgtagg cgccctaagt ttgaagggca tccccctgag tcttggaagt 1020
ggatccttgc accggtcatt ctttatatct gtgaaaggat cctccggttt taccgctccc 1080
agcagaaggt tgtgattacc aaggttgtta tgcacccatc caaagttttg gaattgcaga 1140
tgaacaagcg tggcttcagc atggaagtgg ggcagtatat ctttgttaat tgcccctcaa 1200
tctctctcct ggaatggcat ccttttactt tgacctctgc tccagaggaa gatttcttct 1260
ccattcatat ccgagcagca ggggactgga cagaaaatct cataagggct ttcgaacaac 1320
aatattcacc aattcccagg attgaagtgg atggtccctt tggcacagcc agtgaggatg 1380
ttttccagta tgaagtggct gtgctggttg gagcaggaat tggggtcacc ccctttgctt 1440
ctatcttgaa atccatctgg tacaaattcc agtgtgcaga ccacaacctc aaaacaaaaa 1500
agatctattt ctactggatc tgcagggaga caggtgcctt ttcctggttc aacaacctgt 1560
tgacttccct ggaacaggag atggaggaat taggcaaagt gggttttcta aactaccgtc 1620
tcttcctcac cggatgggac agcaatattg ttggtcatgc agcattaaac tttgacaagg 1680
ccactgacat cgtgacaggt ctgaaacaga aaacctcctt tgggagacca atgtgggaca 1740
atgagttttc tacaatagct acctcccacc ccaagtctgt agtgggagtt ttcttatgtg 1800
gccctcggac tttggcaaag agcctgcgca aatgctgtca ccgatattcc agtctggatc 1860
ctagaaaggt tcaattctac ttcaacaaag aaaatttttg agttatagga ataaggacgg 1920
taatctgcat tttgtctctt tgtatcttca gtaattgagt tataggaata aggacggtaa 1980
tctgcatttt gtctctttgt atcttcagta atttacttgg tctcntcagg tttgancagt 2040
cactttagat aagaatgtgc ctctcaagcc ttgactccct ggtattcttt ttttgattgc 2100
attcaacttc gttacttgag cttcagcaac ttaagaactt ctgaagttct taaagttctg 2160
aanttcttaa agcccatgga tcctttctca gaaaaataac tgtaaatctt tctggacagc 2220
catgactgta gcaaggcttg atagcagaag tttggtggtt canaattata caactaatcc 2280
caggtgattt tatcaattcc agtgttacca tctcctgagt tttggtttgt aatcttttgt 2340
ccctcccacc cccacagaag attttaagta gggtgacttt ttaaataaaa atttattgaa 2400
taattaatga taaaacataa taataaacat aaataataaa caaaattacc gagaacccca 2460
tccccatata acaccaacag tgtacatgtt tactgtcact tttgatatgg tttatccagt 2520
gtgaacagca atttattatt tttgctcatc aaaaaataaa ggattttttt tcacttgaaa 2580
aaaaaaaaaa aaaaaaaaaa aaaaaaaa 2608

## Description

### FIELD OF THE INVENTION

This invention relates, in part, to newly developed assays for detecting, diagnosing, monitoring, staging, prognosticating, imaging and treating cancers, particularly colon cancer.

### BACKGROUND OF THE INVENTION

Cancer of the colon is a highly treatable and often curable disease when localized to the bowel. It is one of the most frequently diagnosed malignancies in the United States as well as the second most common cause of cancer death. Surgery is the primary treatment and results in cure in approximately 50% of patients. However, recurrence following surgery is a major problem and often is the ultimate cause of death.

The prognosis of colon cancer is clearly related to the degree of penetration of the tumor through the bowel wall and the presence or absence of nodal involvement. These two characteristics form the basis for all staging systems developed for this disease. Treatment decisions are usually made in reference to the older Duke's or the Modified Astler-Coller (MAC) classification scheme for staging.

Bowel obstruction and bowel perforation are indicators of poor prognosis in patients with colon cancer. Elevated pretreatment serum levels of carcinoembryonic antigen (CEA) and of carbohydrate antigen 19-9 (CA 19-9) also have a negative prognostic significance.

Age greater than 70 years at presentation is not a contraindication to standard therapies. Acceptable morbidity and mortality, as well as long-term survival, are achieved in this patient population.

Because of the frequency of the disease (approximately 160,000 new cases of colon and rectal cancer per year), the identification of high-risk groups, the demonstrated slow growth of primary lesions, the better survival of early-stage lesions, and the relative simplicity and accuracy of screening tests, screening for colon cancer should be a part of routine care for all adults starting at age 50, especially those with first-degree relatives with colorectal cancer.

Procedures used for detecting, diagnosing, monitoring, staging, and prognosticating colon cancer are of critical importance to the outcome of the patient. For example, patients diagnosed with early colon cancer generally have a much greater five-year survival rate as compared to the survival rate for patients diagnosed with distant metastasized colon cancer. New diagnostic methods which are more sensitive and specific for detecting early colon cancer are clearly needed.

Colon cancer patients are closely monitored following initial therapy and during adjuvant therapy to determine response to therapy and to detect persistent or recurrent disease of metastasis. There is clearly a need for a colon cancer marker which is more sensitive and specific in detecting colon cancer, its recurrence, and progression.

Another important step in managing colon cancer is to determine the stage of the patient's disease. Stage determination has potential prognostic value and provides criteria for designing optimal therapy. Generally, pathological staging of colon cancer is preferable over clinical staging because the former gives a more accurate prognosis. However, clinical staging would be preferred were it at least as accurate as pathological staging because it does not depend on an invasive procedure to obtain tissue for pathological evaluation. Staging of colon cancer would be improved by detecting new markers in cells, tissues, or bodily fluids which could differentiate between different stages of invasion.

Accordingly, there is a great need for more sensitive and accurate methods for the staging of colon cancer in a human to determine whether or not such cancer has metastasized and for monitoring the progress of colon cancer in a human which has not metastasized for the onset of metastasis.

In the present invention, methods are provided for detecting, diagnosing, monitoring, staging, prognosticating, imaging and treating colon cancer via a colon specific gene referred to herein as CSG. For purposes of the present invention, CSG refers, among other things, to native protein expressed by the gene comprising a polynucleotide sequence of SEQ ID NO:1. By "CSG" as used herein it is also meant to be inclusive of splice variants of the polynucleotide of SEQ ID NO: 84. Exemplary CSG splice variants of the present invention are set forth in SEQ ID NO:1 and 2. By "CSG" it is also meant herein polynucleotides which, due to degeneracy in genetic coding, comprise variations in nucleotide sequence as compared to SEQ ID NO:84, SEQ ID NQ: 1 or SEQ ID NO:2 but which still encode the same proteins. An exemplary CSG protein of the present invention encoded by SEQ ID NO:1 is depicted in SEQ ID NO:3. An exemplary CSG protein of the present invention encoded by SEQ ID NO:2 is depicted in SEQ ID NO:4. In the alternative, what is meant by CSG as used herein, means the native mRNA encoded by the gene comprising the polynucleotide sequence of SEQ ID NO:84, SEQ ID NO: 1 or SEQ ID NO:2, levels of the gene comprising the polynucleotide sequence of SEQ ID NO:84, SEQ ID NO: 1 of SEQ ID NO:2, or levels of a polynucleotide which is capable of hybridizing under stringent conditions to the antisense sequence of SEQ ID NO: 84, SEQ ID NO: 1 or SEQ ID NO:2. SEQ ID NO:84 is also referred to as Cln106 while SEQ ID NO:1 and 2 are referred to as splice variants of Cln106.

Other objects, features, advantages and aspects of the present invention will become apparent to those of skill in the art from the following description. It should be understood, however, that the following description and the specific examples, while indicating preferred embodiments of the invention are given by way of illustration only. Various changes and modifications within the spirit and scope of the disclosed invention will become readily apparent to those skilled in the art from reading the following description and from reading the other parts of the present disclosure.

### SUMMARY OF THE INVENTION

Toward these ends, and others, it is an object of the present invention to provide a method for diagnosing the presence of colon cancer by analyzing for changes in levels of CSG in cells, tissues or bodily fluids compared with levels of CSG in preferably the same cells, tissues, or bodily fluid type of a normal human control, wherein a change in levels of CSG in the patient versus the normal human control is associated with colon cancer.

Further provided is a method of diagnosing metastatic colon cancer in a patient having colon cancer which is not known to have metastasized by identifying a human patient suspected of having colon cancer that has metastasized; analyzing a sample of cells, tissues, or bodily fluid from such patient for CSG; comparing the CSG levels in such cells, tissues, or bodily fluid with levels of CSG in preferably the same cells, tissues, or bodily fluid type of a normal human control, wherein an increase in CSG levels in the patient versus the normal human control is associated with colon cancer which has metastasized.

Also provided by the invention is a method of staging colon cancer in a human which has such cancer by identifying a human patient having such cancer; analyzing a sample of cells, tissues, or bodily fluid from such patient for CSG; comparing CSG levels in such cells, tissues, or bodily fluid with levels of CSG in preferably the same cells, tissues, or bodily fluid type of a normal human control sample, wherein an increase in CSG levels in the patient versus the normal human control is associated with a cancer which is progressing or at a more advanced stage and a decrease in the levels of CSG is associated with a cancer which is regressing or at a lower stage or in remission.

Further provided is a method of monitoring colon cancer in a human having such cancer for the onset of metastasis. The method comprises identifying a human patient having such cancer that is not known to have metastasized; periodically analyzing a sample of cells, tissues, or bodily fluids from such patient for CSG; comparing the CSG levels in such cells, tissues, or bodily fluids with levels of CSG in preferably the same cells, tissues, or bodily fluid type of a normal human control sample, wherein an increase in CSG levels in the patient versus the normal human control is associated with a cancer which has metastasized.

Further provided is a method of monitoring the change in stage of colon cancer in a human having such cancer by looking at levels of CSG in a human having such cancer. The method comprises identifying a human patient having such cancer; periodically analyzing a sample of cells, tissues, or bodily fluids from such patient for CSG; comparing the CSG levels in such cells, tissues, or bodily fluids with levels of CSG in preferably the same cells, tissues, or bodily fluid type of a normal human control sample, wherein an increase in CSG levels in the patient versus the normal human control is associated with a cancer which is progressing and a decrease in the levels of CSG is associated with a cancer which is regressing or in remission.

Further provided are methods of designing new therapeutic agents targeted to a CSG for use in imaging and treating colon cancer. For example, in one embodiment, therapeutic agents such as antibodies targeted against CSG or fragments of such antibodies can be used to treat, detect or image localization of CSG in a patient for the purpose of detecting or diagnosing a disease or condition. In this image embodiment, an increase in the amount of labeled antibody detected as compared to normal tissue would be indicative of tumor metastases or growth. Such antibodies can be polyclonal, monoclonal, or omniclonal or prepared by molecular biology techniques. The term "antibody", as used herein and throughout the instant specification is also meant to include aptamers and single-stranded oligonucleotides such as those derived from an in vitro evolution protocol referred to as SELEX and well known to those skilled in the art. Antibodies can be labeled with a variety of detectable labels and therapeutic agents including, but not limited to, radioisotopes and paramagnetic metals. Therapeutic agents such as small molecules and antibodies which decrease the concentration and/or activity of CSG can also be used in the treatment of diseases characterized by overexpression of CSG. Such agents can be readily identified in accordance with teachings herein.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### DETAILED DESCRIPTION OF THE INVENTION

The present invention relates to, inter alia, diagnostic assays and methods, both quantitative and qualitative for detecting, diagnosing, monitoring, staging and prognosticating cancer, and in particular colon cancer, by comparing levels of CSG in a human patient with those of CSG in a normal human control. It has now been found the CSG levels are elevated in colon cancer tissue as compared to normal tissue. For purposes of the present invention, CSG refers, among other things, to native protein expressed by the gene comprising a polynucleotide sequence of SEQ ID NO:84. By "CSG" as used herein it is also meant to be inclusive of splice variants of the polynucleotide of SEQ ID NO: 84. Exemplary CSG splice variant of the present invention are set forth in SEQ ID NO:1 and SEQ ID NO:2. By "CSG" it is also meant herein polynucleotides which, due to degeneracy in genetic coding, comprise variations in nucleotide sequence as compared to SEQ ID NO:84, SEQ ID NO: 1 or SEQ ID NO:2 but which still encode the same proteins or fragments thereof. An exemplary CSG protein of the present invention encoded by SEQ ID NO:1 is depicted in SEQ ID NO:3. An exemplary CSG protein of the present invention encoded by SEQ ID NO:2 is depicted in SEQ ID NO:4. In the alternative, what is meant by CSG as used herein, means the native mRNA encoded by the gene comprising the polynucleotide sequence of SEQ ID NO:84, SEQ ID NO: 1 or SEQ ID NO:2, levels of the gene comprising the polynucleotide sequence of SEQ ID NO:84, SEQ ID NO: 1 of SEQ ID NO:2, or levels of a polynucleotide which is capable of hybridizing under stringent conditions to the antisense sequence of SEQ ID NO:84, SEQ ID NO: 1 or SEQ ID NO:2. SEQ ID NO:84 is also referred to as Cln106 while SEQ ID NO: 1 and SEQ ID NO:2 are referred to as splice variants of Cln106. Levels of CSG are preferably determined in at least one of cells, tissues and/or bodily fluids, including determination of normal and abnormal levels. Thus, for instance, a diagnostic assay in accordance with the invention for diagnosing overexpression of CSG protein compared to normal control bodily fluids, cells, or tissue samples may be used to diagnose the presence of colon cancer.

All the methods of the present invention may optionally include determining the levels of other cancer markers as well as CSG. Other cancer markers, in addition to CSG, useful in the present invention are known to those of skill in the art.

### Diagnostic Assays

The present invention provides methods for diagnosing the presence of cancer, and in particular colon cancer, by analyzing for changes in levels of CSG in cells, tissues or bodily fluids compared with levels of CSG in cells, tissues or bodily fluids of preferably the same type from a normal human control, wherein an increase in levels of CSG in the patient versus the normal human control is associated with the presence of colon cancer.

Without limiting the instant invention, typically, for a quantitative diagnostic assay a positive result indicating the patient being tested has cancer is one in which cells, tissues or bodily fluid levels of the cancer marker, such as CSG, are at least two times higher, and most preferably are at least five times higher, than in preferably the same cells, tissues or bodily fluid of a normal human control.

The present invention also provides a method of diagnosing metastatic colon cancer in a patient having colon cancer which has not yet metastasized for the onset of metastasis. In the method of the present invention, a human cancer patient suspected of having colon cancer which may have metastasized (but which was not previously known to have metastasized) is identified. This is accomplished by a variety of means known to those of skill in the art.

In the present invention, determining the presence of CSG levels in cells, tissues or bodily fluid, is particularly useful for discriminating between colon cancer which has not metastasized and colon cancer which has metastasized. Existing techniques have difficulty discriminating between colon cancer which has metastasized and colon cancer which has not metastasized and proper treatment selection is often dependent upon such knowledge.

In the present invention, the cancer marker levels measured in such cells, tissues or bodily fluid is CSG, and are compared with levels of CSG in preferably the same cells, tissues or bodily fluid type of a normal human control. That is, if the cancer marker being observed is CSG in serum, this level is preferably compared with the level of CSG in serum of a normal human control. An increase in the CSG in the patient versus the normal human control is associated with colon cancer which has metastasized.

Without limiting the instant invention, typically, for a quantitative diagnostic assay a positive result indicating the cancer in the patient being tested or monitored has metastasized is one in which cells, tissues or bodily fluid levels of the cancer marker, such as CSG, are at least two times higher, and most preferably are at least five times higher, than in preferably the same cells, tissues or bodily fluid of a normal patient.

Normal human control as used herein includes a human patient without cancer and/or non cancerous samples from the patient; in the methods for diagnosing or monitoring for metastasis, normal human control may preferably also include samples from a human patient that is determined by reliable methods to have colon cancer which has not metastasized. Staging

The invention also provides a method of staging colon cancer in a human patient. The method comprises identifying a human patient having such cancer and analyzing cells, tissues or bodily fluid from such human patient for CSG. The CSG levels determined in the patient are then compared with levels of CSG in preferably the same cells, tissues or bodily fluid type of a normal human control, wherein an increase in CSG levels in the human patient versus the normal human control is associated with a cancer which is progressing or at a higher stage and a decrease in the levels of CSG (but generally still increased over true normal levels) is associated with a cancer which is regressing or at a lower stage or in remission,

### Monitoring

Further provided is a method of monitoring colon cancer in a human patient having such cancer for the onset of metastasis. The method comprises identifying a human patient having such cancer that is not known to have metastasized; periodically analyzing cells, tissues or bodily fluid from such human patient for CSG; and comparing the CSG levels determined in the human patient with levels of CSG in preferably the same cells, tissues or bodily fluid type of a normal human control, wherein an increase in CSG levels in the human patient versus the normal human control is associated with a cancer which has metastasized. In this method, normal human control samples may also include prior samples from the same patient.

Further provided by this invention is a method of monitoring the change in stage of colon cancer in a human patient having such cancer. The method comprises identifying a human patient having such cancer; periodically analyzing cells, tissues or bodily fluid from such human patient for CSG; and comparing the CSG levels determined in the human patient with levels of CSG in preferably the same cells, tissues or bodily fluid type of a normal human control, wherein an increase in CSG levels in the human patient versus the normal human control is associated with a cancer which is progressing in stage and a decrease in the levels of CSG is associated with a cancer which is regressing in stage or in remission. In this method, normal human control samples may also include prior patient samples.

Monitoring a patient for onset of metastasis is periodic and preferably done on a quarterly basis. However, this may be done more or less frequently depending on the cancer, the particular patient, and the stage of the cancer.

### Prognostic Testing and Clinical Trial Monitoring

The methods described herein can further be utilized as prognostic assays to identify subjects having or at risk of developing a disease or disorder associated with increased levels of CSG. The present invention provides a method in which a test sample is obtained from a human patient and a CSG is detected. The presence of higher CSG levels as compared to normal human controls is diagnostic for the human patient being at risk for developing cancer, specifically colon cancer.

The effectiveness of therapeutic agents to decrease expression or activity of the CSG of the invention can also be monitored by analyzing levels of expression of the CSG in a human patient, e.g. during treatment, in clinical trials or in *in vitro* screening assays such as in human cells. In this way, the CSG expression pattern can serve as a marker, indicative of the physiological response of the human patient, or cells as the case may be, to the agent being tested or being used to treat the patient.

### Detection of genetic lesions or mutations

The methods of the present invention can also be used to detect genetic lesions or mutations in a CSG, thereby determining if a human with the genetic lesion is at risk for colon cancer or has colon cancer. Genetic lesions can be detected, for example, by ascertaining the existence of a deletion and/or addition and/or substitution of one or more nucleotides from the CSG of this invention, a chromosomal rearrangement of a CSG, aberrant modification of CSG (such as of the methylation pattern of the genomic DNA), the presence of a non-wild type splicing pattern of a mRNA transcript of a CSG, allelic loss of a CSG, and/or inappropriate post-translational modification of a CSG protein. Methods to detect such lesions in the CSG of this invention are known to those of skill in the art.

### Assay Techniques

Assay techniques that can be used to determine levels of gene expression (including protein levels), such as CSG of the present invention, in a sample derived from a patient are well known to those of skill in the art. Such assay methods include, without limitation, radioimmunoassays, reverse transcriptase PCR (RT-PCR) assays, immunohistochemistry assays, *in situ* hybridization assays, competitive-binding assays, Western Blot analyses, ELISA assays and proteomic approaches, two-dimensional gel electrophoresis (2D electrophoresis) and non-gel based approaches such as mass spectrometry or protein interaction profiling. Among these, ELISAs are frequently preferred to diagnose a gene's expressed protein in biological fluids.

An ELISA assay initially comprises preparing an antibody, if not readily available from a commercial source, specific to CSG, preferably a monoclonal antibody. In addition a reporter antibody generally is prepared which binds specifically to CSG. The reporter antibody is attached to a detectable reagent such as radioactive, fluorescent or enzymatic reagent, for example horseradish peroxidase enzyme or alkaline phosphatase.

To carry out the ELISA, antibody specific to CSG is incubated on a solid support, e.g. a polystyrene dish, that binds the antibody. Any free protein binding sites on the dish are then covered by incubating with a non-specific protein such as bovine serum albumin. Next, the sample to be analyzed is incubated in the dish, during which time CSG binds to the specific antibody attached to the polystyrene dish. Unbound sample is washed out with buffer. A reporter antibody specifically directed to CSG and linked to a detectable reagent such as horseradish peroxidase is placed in the dish resulting in binding of the reporter antibody to any monoclonal antibody bound to CSG. Unattached reporter antibody is then washed out. Reagents for peroxidase activity, including a colorimetric substrate are then added to the dish. Immobilized peroxidase, linked to CSG antibodies, produces a colored reaction product. The amount of color developed in a given time period is proportional to the amount of CSG protein present in the sample. Quantitative results typically are obtained by reference to a standard curve .

A competition assay can also be employed wherein antibodies specific to CSG are attached to a solid support and labeled CSG and a sample derived from the host are passed over the solid support. The amount of label detected which is attached to the solid support can be correlated to a quantity of CSG in the sample.

Using all or a portion of a nucleic acid sequence of a CSG of the present invention as a hybridization probe, nucleic acid methods can also be used to detect levels of CSG mRNA as a marker for colon cancer. Polymerase chain reaction (PCR) and other nucleic acid methods, such as ligase chain reaction (LCR) and nucleic acid sequence based amplification (NASBA), can be used to detect cells for diagnosis and monitoring of various malignancies. For example, reverse-transcriptase PCR (RT-PCR) is a powerful technique which can be used to detect the presence of a specific mRNA population in a complex mixture of thousands of other mRNA species. In RT-PCR, an mRNA species is first reverse transcribed to complementary DNA (cDNA) with use of the enzyme reverse transcriptase; the cDNA is then amplified as in a standard PCR reaction. RT-PCR can thus reveal by amplification the presence of a single species of mRNA. Accordingly, if the mRNA is highly specific for the cell that produces it, RT-PCR can be used to identify the presence and/or absence of a specific type of cell.

Hybridization to clones or oligonucleotides arrayed on a solid support (i.e. gridding) can be used to both detect the expression of and quantitate the level of expression of the gene. In this approach, all or a portion of a cDNA encoding the CSG gene is fixed to a substrate. The substrate may be of any suitable type including but not limited to glass, nitrocellulose, nylon or plastic. At least a portion of the DNA encoding the CSG gene is attached to the substrate and then incubated with the analyte, which may be RNA or a complementary DNA (cDNA) copy of the RNA, isolated from the tissue of interest. Hybridization between the substrate bound DNA and the analyte can be detected and quantitated by several means including but not limited to radioactive labeling or fluorescence labeling of the analyte or a secondary molecule designed to detect the hybrid. Quantitation of the level of gene expression can be done by comparison of the intensity of the signal from the analyte compared with that determined from known standards. The standards can be obtained by in vitro transcription of the target gene, quantitating the yield, and then using that material to generate a standard curve.

Of the proteomic approaches, 2D electrophoresis is a technique well known to those skilled in the art. Isolation of individual proteins from a sample such as serum is accomplished using sequential separation of proteins by different characteristics usually on polyacrylamide gels. First, proteins are separated by size using an electric current. The current acts uniformly on all proteins, so smaller proteins move farther on the gel than larger proteins. The second dimension applies a current perpendicular to the first and separates proteins not on the basis of size but on the specific electric charge carried by each protein. Since no two proteins with different sequences are identical on the basis of both size and charge, the result of a 2D separation is a square gel in which each protein occupies a unique spot. Analysis of the spots with chemical or antibody probes, or subsequent protein microsequencing can reveal the relative abundance of a given protein and the identity of the proteins in the sample.

The above tests can be carried out on samples derived from a variety of cells, bodily fluids and/or tissue extracts such as homogenates or solubilized tissue obtained from a patient. Tissue extracts are obtained routinely from tissue biopsy and autopsy material. Bodily fluids useful in the present invention include blood, urine, saliva or any other bodily secretion or derivative thereof. By blood it is meant to include whole blood, plasma, serum or any derivative of blood.

### In Vivo Targeting of CSG/Colon Cancer Therapy

Identification of this CSG is also useful in the rational design of new therapeutics for imaging and treating cancers, and in particular colon cancer. For example, in one embodiment, antibodies which specifically bind to the CSG can be raised and used *in vivo* in patients suspected of suffering from colon cancer associated with increased levels of CSG. Antibodies which specifically bind the CSG can be injected into a patient suspected of having colon cancer for diagnostic and/or therapeutic purposes. Thus, another aspect of the present invention provides for a method for preventing the onset and treatment of colon cancer in a human patient in need of such treatment by administering to the patient an effective amount of an antibody to CSG. By "effective amount" it is meant the amount or concentration of antibody needed to bind to the target antigens expressed on the tumor to cause tumor shrinkage for surgical removal, or disappearance of the tumor. The binding of the antibody to the overexpressed CSG is believed to cause the death of the cancer cell expressing such CSG. The antibodies can be administered alone or with other therapeutic agents known to those in the art.

The preparation and use of antibodies for *in vivo* diagnosis and treatment is well known in the art. For example, antibody-chelators labeled with Indium-111 have been described for use in the radioimmunoscintographic imaging of carcinoembryonic antigen expressing tumors (Sumerdon et al. Nucl. Med. Biol. 1990 17:247-254). In particular, these antibody-chelators have been used in detecting tumors in patients suspected of having recurrent colorectal cancer (Griffin et al. J. Clin. Onc. 1991 9:631-640). Antibodies with paramagnetic ions as labels for use in magnetic resonance imaging have also been described (Lauffer, R.B. Magnetic Resonance in Medicine 1991 22:339-342). Antibodies directed against CSG can be used in a similar manner. Labeled antibodies which specifically bind CSG can be injected into patients suspected of having colon cancer for the purpose of diagnosing, monitoring or staging of the disease status of the patient. The label used will be selected in accordance with the imaging modality to be used. For example, radioactive labels such as Indium-111, Technetium-99m or Iodine-131 can be used for planar scans or single photon emission computed tomography (SPECT). Positron emitting labels such as Fluorine-19 can be used in positron emission tomography. Paramagnetic ions such as Gadlinium (III) or Manganese (II) can be used in magnetic resonance imaging (MRI). Presence of the label, as compared to imaging of normal tissue, permits determination of the spread of the cancer. The amount of label within an organ or tissue also allows determination of the presence or absence of cancer in that organ or tissue.

Antibodies which can be used in in vivo methods include polyclonal, monoclonal and omniclonal antibodies and antibodies prepared via molecular biology techniques. Antibody fragments and aptamers and single-stranded oligonucleotides such as those derived from an *in vitro* evolution protocol referred to as SELEX and well known to those skilled in the art can also be used. The present invention also includes chimeric, single chain, and humanized antibodies, as well as Fab fragments, or the product of an Fab expression library. Various procedures known in the art may be used for the production of such antibodies and fragments.

A variety of methods for antibody production are set forth in Current Protocols, chapter 2.

For example, cells expressing a CSG polypeptide of the present invention can be administered to an animal to induce the production of sera containing polyclonal antibodies. In a preferred method, a preparation of the secreted protein is prepared and purified to render it substantially free of natural contaminants. This preparation is then introduced into an animal in order to produce polyclonal antisera of greater specific activity. The antibody obtained will bind with the CSG polypeptide itself. In this manner, even a sequence encoding only a fragment of the CSG polypeptide can be used to generate antibodies binding the whole native polypeptide. Such antibodies can then be used to isolate the CSG polypeptide from tissue expressing that CSG polypeptide.

Alternatively, monoclonal antibodies can be prepared. Examples of techniques for production of monoclonal antibodies include, but are not limited to, the hybridoma technique (Kohler, G. and Milstein, C., Nature 256: 495-497 (1975), the trioma technique, the human B-cell hybridoma technique (Kozbor et al., Immunology Today 4: 72 (1983) and (Cole et al., pg. 77-96 in MONOCLONAL ANTIBODIES AND CANCER THERAPY, Alan R. Liss, Inc. (1985). The EBV-hybridoma technique is useful in production of human monoclonal antibodies.

Hybridoma technologies have also been described by Khler et al. (Eur. J. Immunol. 6: 511 (1976)) Khler et al. (Eur. J.Immunol. 6: 292 (1976)) and Hammerling et al. (in: Monoclonal Antibodies and T-Cell Hybridomas, Elsevier, N. Y., pp. 563-681 (1981)). In general, such procedures involve immunizing an animal (preferably a mouse) with CSG polypeptide or, more preferably, with a secreted CSG polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin. The splenocytes of such mice are extracted and fused with a suitable myeloma cell line. Any suitable myeloma cell line may be employed in accordance with the present invention; however, it is preferable to employ the parent myeloma cell line (SP20), available from the ATCC. After fusion, the resulting hybridoma cells are selectively maintained in HAT medium, and then cloned by limiting dilution as described by Wands et al. (Gastroenterology 80: 225-232 (1981).). The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein specific antibody and can be used to immunize an animal to induce formation of further protein-specific antibodies.

Techniques described for the production of single chain antibodies (U.S. Patent 4,946,778) can also be adapted to produce single chain antibodies to immunogenic polypeptide products of this invention. Also, transgenic mice, as well as other nonhuman transgenic animals, may be used to express humanized antibodies to immunogenic polypeptide products of this invention.

It will be appreciated that Fab, F(ab')2 and other fragments of the antibodies of the present invention may also be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art (See, for review, Morrison, Science 229: 1202 (1985); Oi et al., BioTechniques 4: 214 (1986); Cabilly et al., U.S. Patent 4,816,567; Taniguchi et al., EP 171496; Morrison et al., EP 173494; Neuberger et al., WO 8601533; Robinson et al., WO 8702671; Boulianne et al., Nature 312: 643 (1984); Neuberger et al., Nature 314: 268 (1985).)

The above-described antibodies may be employed to isolate or to identify clones expressing CSG polypeptides or purify CSG polypeptides of the present invention by attachment of the antibody to a solid support for isolation and/or purification by affinity chromatography. As discussed in more detail *supra*, antibodies specific against a CSG may also be used to image tumors, particularly cancer of the colon, in patients suffering from cancer. Such antibodies may also be used therapeutically to target tumors expressing a CSG.

The following Table depicts areas of the CSG of SEQ ID NO:3 identified as preferred epitopes of a CSG to which antibodies of the present invention are raised.

**TABLE 1: Preferred Antibody binding sites of SEQ ID NO:3**

| Asn_Glycosylation N~(P)(S,T)~(P) N~P(T)~P | | | |
|---|---|---|---|
| 63 | LCLNF (SEQ ID NO:5) | NSTL (SEQ ID NO:6) | ILLPV (SEQ ID NO:7 |
| N~P(T)~P | | | |
| 97 | KQLDH (SEQ ID NO:8) | NLTF (SEQ ID NO:9) | HKLVA (SEQ ID NO:10) |
| N~P(T)~P | | | |
| 162 | PIQSR (SEQ ID NO:11) | NTTV (SEQ ID NO:12) | EYVTF (SEQ ID NO:13) |
| N~P(S)~P | | | |
| 236 | TEESM (SEQ ID NO:14) | NESH (SEQ ID NO:15) | PRKCA (SEQ ID NO:16) |

| Ck2_Phospho_Site (S,T)x2(D,E) (S)x{2}(E) | | | |
|---|---|---|---|
| 145 | ILSSL (SEQ ID NO:17) | SHDE (SEQ ID NO:18) | KKGGS (SEQ ID NO:19) |
| (T)x{2}(E) | | | |
| 163 | IQSRN (SEQ ID NO:20) | TTVE (SEQ ID NO:21) | YVTFT (SEQ ID NO:22) |
| (S)x{2}(E) | | | |
| 191 | ILMVT (SEQ ID NO:23) | SATE (SEQ ID NO:24) | FIRRS (SEQ ID NO:25) |
| (S)x{2}(E) | | | |
| 199 | EFIRR (SEQ ID NO:26) | SYFE (SEQ ID NO:27) | VFMYT (SEQ ID NO:28) |
| (S)x{2}(E) | | | |
| 234 | GQTEE (SEQ ID NO:29) | SMNE (SEQ ID NO:30) | SHPRK (SEQ ID NO:31) |
| (S)x{2}(E) | | | |
| 333 | NCPST (SEQ ID NO:32) | SLLE (SEQ ID NO:33) | WHPFT (SEQ ID NO:34) |
| (S)x{2}(E) | | | |
| 344 | PFTLT (SEQ ID NO:35) | SAPE (SEQ ID NO:36) | EDFFS (SEQ ID NO:37) |
| (T)x{2}(E) | | | |
| 387 | DGPFG (SEQ ID NO:38) | TASE (SEQ ID NO:39) | DVFQY (SEQ ID NO:40) |
| (T)x{2}(E) | | | |
| 453 | FNNLL (SEQ ID NO:41) | TSLE (SEQ ID NO:42) | QEMEE (SEQ ID NO:43) |
| (T)x{2}(D) | | | |
| 475 | YRLFL (SEQ ID NO:44) | TGWD (SEQ ID NO:45) | SNIVG (SEQ ID NO:46) |
| (S)x{2}(D) | | | |
| 548 | CCHRY (SEQ ID NO:47) | SSLD (SEQ ID NO:48) | PRKVQ (SEQ ID NO:49) |

| Myristyl G~(E,D,R,K,H,P,F,Y,W)x2 (S,T,A,G,C,N)~(P) G-(E,D,R,K,H,P,F,Y,W)x{2}(A)~P | | | |
|---|---|---|---|
| 47 | TRKIL (SEQ ID NO:50) | GSTLAC (SEQ ID NO:51) | ARASA (SEQ ID NO:52) |
| G~(E,D,R,K,H,P,F,Y,W)x{2}(S)~P | | | |
| 135 | RQATD (SEQ ID NO:53) | GSLASI (SEQ ID NO:54) | LSSLS (SEQ ID NO:55) |
| G-(E,D,R,K,H,P,F,Y,W)x{2}(T)<P | | | |
| 178 | VAGLT (SEQ ID NO:56) | GVIMTI (SEQ ID NO:57) | ALILM (SEQ ID NO:58) |
| G~(E,D,R,K,H,P,F,Y,W)x{2}(G)~P | | | |
| 225 | IHGIG (SEQ ID NO:59) | GIVRGQ (SEQ ID NO:60) | TEESM (SEQ ID NO:61) |
| G~(E,D,R,K,H,P,F,Y,W)x{2}(G)~P | | | |
| 402 | VAVLV (SEQ ID NO:62) | GAGIGV (SEQ ID NO:63) | TPFAS (SEQ ID NO:64) |
| G-(E,D,R,K,H,P,F,Y,W)x{2}(C)~P | | | |
| 527 | PKSVV (SEQ ID NO:65) | GVFLCG (SEQ ID NO:66) | PRTLA (SEQ ID NO:67) |
| Pkc_Phospho_Site (S,T)x(R,K)(T)x(K) | | | |
| 42 | DKYYY (SEQ ID NO:68) | TRK | ILGST (SEQ ID NO:69) |
| (T)x(R) | | | |
| 89 | SFCSR (SEQ ID NO:70) | TLR | KQLDH (SEQ ID NO:71) |
| (S)x(K) | | | |
| 269 | GHPPE (SEQ ID NO:72) | SWK | WILAP (SEQ ID NO:73) |
| (T)x(K) | | | |
| 430 | DHNLK (SEQ ID NO:74) | TKK | IYFYW (SEQ ID NO:75) |
| (S) x (R) | | | |
| 539 | RTLAK (SEQ ID NO:76) | SLR | KCCHR (SEQ ID NO:77) |

| Tyr_Phospho_Site(R,K)x{2,3}(D,E)x{2,3}Y (R)x{3}(E)x{3}Y | | | |
|---|---|---|---|
| 198 | TEFIR (SEQ ID NO:78) | RSYFEVFWY (SEQ ID NO:79) | THHLF (SEQ ID NO:80) |
| (R)x{2}(E)x{2}Y | | | |
| 367 | TENLI (SEQ ID NO:81) | RAFEQQY (SEQ ID NO:82) | SPIPR (SEQ ID NO:83) |

### Screening Assays

The present invention also provides methods for identifying modulators which bind to CSG protein of the invention or have a modulatory effect on the expression or activity of CSG protein of this invention. Modulators which decrease the expression or activity of CSG protein of the invention are believed to be useful in treating colon cancer. Such screening assays are known to those of skill in the art and include, without limitation, cell-based assays and cell free assays.

Small molecules predicted via computer imaging to specifically bind to regions of CSG can also be designed, synthesized and tested for use in the imaging and treatment of colon cancer. Further, libraries of molecules can be screened for potential anticancer agents by assessing the ability of the molecule to bind to the CSG identified herein. Molecules identified in the library as being capable of binding to CSG are key candidates for further evaluation for use in the treatment of colon cancer. In a preferred embodiment, these molecules will downregulate expression and/or activity of CSG in cells.

### Adoptive Immunotherapy and Vaccines

Adoptive immunotherapy of cancer refers to a therapeutic approach in which immune cells with an antitumor reactivity are administered to a tumor-bearing host, with the aim that the cells mediate either directly or indirectly, the regression of an established tumor. Transfusion of lymphocytes, particularly T lymphocytes, falls into this category and investigators at the National Cancer Institute (NCI) have used autologous reinfusion of peripheral blood lymphocytes or tumor-infiltrating lymphocytes (TIL), T cell cultures from biopsies of subcutaneous lymph nodules, to treat several human cancers (Rosenberg, S. A., U.S. Patent No. 4,690,914, issued Sep. 1, 1987, Rosenberg, S. A., et al., 1988, N. England J. Med. 319:1676-1680).

The present invention relates to compositions and methods of adoptive immunotherapy for the prevention and/or treatment of primary and metastatic colon cancer in humans using macrophages sensitized to the antigenic CSG molecule of this invention, with or without non-covalent complexes of heat shock protein (hsp). Antigenicity or immunogenicity of the CSG of the invention is readily confirmed by the ability of the CSG protein or a fragment thereof to raise antibodies or educate naive effector cells, which in turn lyse target cells expressing the antigen (or epitope).

Cancer cells are, by definition, abnormal and contain proteins which should be recognized by the immune system as foreign since they are not present in normal tissues. However, the immune system often seems to ignore this abnormality and fails to attack tumors. The foreign CSG protein of this invention, that are produced by the cancer cells can be used to reveal their presence. The CSG is broken into short fragments, called tumor antigens, which are displayed on the surface of the cell. These tumor antigens are held or presented on the cell surface by molecules called MHC, of which there are two types: class I and II. Tumor antigens in association with MHC class I molecules are recognized by cytotoxic T cells while antigen-MHC class II complexes are recognized by a second subset of T cells called helper cells. These cells secrete cytokines which slow or stop tumor growth and help another type of white blood cell, B cells, to make antibodies against the tumor cells.

In adoptive immunotherapy, T cells or other antigen presenting cells (APCs) are stimulated outside the body (*ex vivo*), using the tumor specific CSG antigens of the present invention. The stimulated cells are then reinfused into the patient where they attack the cancerous cells. Research has shown that using both cytotoxic and helper T cells is far more effective than using either subset alone. Additionally, the CSG antigen may be complexed with heat shock proteins to stimulate the APCs as described in U.S. Patent No. 5,985,270.

The APCs can be selected from among those antigen presenting cells known in the art, including but not limited to macrophages, dendritic cells, B lymphocytes, and a combination thereof, and are preferably macrophages. In a preferred use, wherein cells are autologous to the individual, autologous immune cells such as lymphocytes, macrophages or other APCs are used to circumvent the issue of whom to select as the donor of the immune cells for adoptive transfer. Another problem circumvented by use of autologous immune cells is graft versus host disease which can be fatal if unsuccessfully treated.

In adoptive immunotherapy with gene therapy, DNA of the CSG of the invention can be introduced into effector cells similarly as in conventional gene therapy. This can enhance the cytotoxicity of the effector cells to tumor cells as they have been manipulated to produce the antigenic protein resulting in improvement of the adoptive immunotherapy.

CSG antigens of this invention are also useful as components of colon cancer vaccines. The vaccine comprises an immunogenically stimulatory amount of a CSG antigen of the present invention. Immunogenically stimulatory amount refers to that amount of antigen that is able to invoke the desired immune response in the recipient for the amelioration, or treatment of colon cancer. Effective amounts may be determined empirically by standard procedures well known to those skilled in the art.

The CSG antigen can be provided in any one of a number of vaccine formulations which are designed to induce the desired type of immune response, e.g., antibody and/or cell mediated. Such formulations are known in the art and include, but are not limited to, formulations such as those described in U.S. Patent 5,585,103. Vaccine formulations of the present invention used to stimulate immune responses can also include pharmaceutically acceptable adjuvants.

## Claims

1. A method for diagnosing the presence of colon cancer in a patient comprising:
(a) determining levels of CSG in cells, tissues or bodily fluids in a patient; and
(b) comparing the determined levels of CSG with levels of CSG in cells, tissues or bodily fluids from a normal human control, wherein a change in determined levels of CSG in said patient versus normal human control is associated with the presence of colon cancer.

2. A method of diagnosing metastases of colon cancer in a patient comprising:
(a) identifying a patient having colon cancer that is not known to have metastasized;
(b) determining CSG levels in a sample of cells, tissues, or bodily fluid from said patient; and
(c) comparing the determined CSG levels with levels of CSG in cells, tissue, or bodily fluid of a normal human control, wherein an increase in determined CSG levels in the patient versus the normal human control is associated with a cancer which has metastasized.

3. A method of staging colon cancer in a patient having colon cancer comprising:
(a) identifying a patient having colon cancer;
(b) determining CSG levels in a sample of cells, tissue, or bodily fluid from said patient; and
(c) comparing determined CSG levels with levels of CSG in cells, tissues, or bodily fluid of a normal human control, wherein an increase in determined CSG levels in said patient versus the normal human control is associated with a cancer which is progressing and a decrease in the determined CSG levels is associated with a cancer which is regressing or in remission.

4. A method of monitoring colon cancer in a patient for the onset of metastasis comprising:
(a) identifying a patient having colon cancer that is not known to have metastasized;
(b) periodically determining levels of CSG in samples of cells, tissues, or bodily fluid from said patient, and
(c) comparing the periodically determined CSG levels with levels of CSG in cells, tissues, or bodily fluid of a normal human control, wherein an increase in any one of the periodically determined CSG levels in the patient versus the normal human control is associated with a cancer which has metastasized.

5. A method of monitoring a change in stage of colon cancer in a patient comprising:
(a) identifying a patient having colon cancer;
(b) periodically determining levels of CSG in cells, tissues, or bodily fluid from said patient; and
(c) comparing the periodically determined CSG levels with levels of CSG in cells, tissues, or bodily fluid of a normal human control, wherein an increase in any one of the periodically determined CSG levels in the patient versus the normal human control is associated with a cancer which is progressing in stage and a decrease is associated with a cancer which is regressing in stage or in remission.

6. A method of identifying potential therapeutic agents for use in imaging and treating colon cancer comprising screening molecules for an ability to bind to CSG or decrease expression of CSG relative to CSG in the absence of the agent wherein the ability of a molecule to bind to CSG or decrease expression of CSG is indicative of the molecule being useful in imaging and treating colon cancer.

7. The method of claim 1, 2, 3, 4, 5 or 6 wherein the CSG comprises SEQ ID NO:84, SEQ ID NO:1 or SEQ ID NO:2 or a polypeptide encoded thereby.

8. The method of claim 7 wherein the polypeptide comprises SEQ ID NO:3 or SEQ ID NO:4.

9. An antibody which specifically binds a polypeptide encoded by SEQ ID NO:84, SEQ ID NO: 1 or SEQ ID NO:2.

10. The antibody of claim 9 which binds a polypeptide comprising SEQ ID NO:3 or 4 or an epitope thereof.

11. The antibody of claim 10 wherein the epitope is set forth in Table 1.

12. A method of imaging colon cancer in a patient comprising administering to the patient an antibody of claim 9.

13. The method of claim 12 wherein said antibody is labeled with paramagnetic ions or a radioisotope.

14. A method of treating colon cancer in a patient comprising administering to the patient a molecule which downregulates expression or activity of CSG.

15. A method of inducing an immune response against a target cell expressing CSG comprising delivering to a human patient an immunogenically stimulatory amount of a CSG protein so that an immune response is mounted against the target cell.
